(19)

Europäisches
Patentamt
European
Patent Office
Office européen
des brevets

(11) **EP 2 311 937 A1**

(12) **EUROPEAN PATENT APPLICATION**

(43) Date of publication:
**20.04.2011 Bulletin 2011/16**

(21) Application number: **10012875.0**

(22) Date of filing: **06.12.2005**

(51) Int Cl.:
*C12N 1/20* (2006.01)        *C12N 1/36* (2006.01)
*A23K 1/16* (2006.01)        *A23K 1/18* (2006.01)
*C12Q 1/04* (2006.01)        *C12R 1/15* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.12.2004 US 633611 P**

(62) Document number(s) of the earlier application(s) in accordance with Art. 76 EPC:
**05857363.5 / 1 831 349**

(71) Applicants:
• **Sage Biosciences Inc.**
  **Calgary AB T2X 1K2 (CA)**
• **Rode, Lyle M.**
  **Lethbridge AB T1K 3J5 (CA)**
• **Newbold, James C.**
  **Dyfed SY24 5DG, Wales (GB)**

(72) Inventors:
• **Rode, Lyle M.**
  **Lethbridge, AB T1K 3J5 (CA)**
• **Newbold, James C.**
  **Dyfed SY24 5DG (GB)**

(74) Representative: **Wright, Simon Mark**
  **J.A. Kemp & Co.**
  **14 South Square**
  **Gray's Inn**
  **London WC1R 5JJ (GB)**

Remarks:
This application was filed on 01-10-2010 as a divisional application to the application mentioned under INID code 62.

(54) **Method of growing bacteria to deliver bioactive compounds to the intestine of ruminants**

(57) Methods for increasing the resistance to rumen inactivation of a cultured Gram positive bacteria strain useful for gastrointestinal delivery of bioactive compounds to ruminants, which includes the steps of growing a culture of the bacteria strain through at least one passage in a growth medium containing an amount of lysozyme effective to induce the growth of bacterial cell walls resistant to protozoal predation; and recovering the bacteria strain from the lysozyme-containing medium. Rumen-bypass feed supplements produced by the inventive method are also disclosed, as well as methods for supplementing the diet of a ruminant with the rumen bypass feed supplements and an in vitro method for evaluating the resistance of Gram positive bacteria strains to rumen inactivation in vivo.

FIG. 4

EP 2 311 937 A1

**Description**

CROSS-REFERENCE TO RELATED APPLICATION

The present invention claims priority benefit under 35 U.S.C. §119(e) of U.S. Provisional Application Serial No. 60/633,611 file December 6, 2004, the disclosure of which is incorporated herein by reference.

TECHNICAL FIELD

This invention relates to a method of identifying microorganisms useful for the gastrointestinal delivery of bioactive compounds to ruminants that are inherently resistant to inactivation within the rumen, as well as a method of growing the less inactivation-resistant useful microorganisms so that they are more resistant to inactivation within the rumen. The microorganisms, when they are orally administered to ruminants, are capable of delivering whole cells gastrointestinally, and the nutrients and bioactive compounds contained within the cells, to ruminants. The present invention also includes the micro-organisms grown more resistant to inactivation in the rumen that are useful for the gastrointestinal delivery of bioactive compounds to ruminants and methods for supplementing the diets of ruminants therewith.

BACKGROUND ART

Probiotic cultures based on Bifidobacterium, Propionibacerium and Lactobacillus are increasingly being used to maintain intestinal function in monogastric farm animals and humans. Claimed benefits include increased digestibility, improved immune function and a reduction in gastrointestinal upsets. Although probiotics, with yeast and fungal probiotics as prime examples, are used in ruminants, the difficulty of ensuring that probiotics pass through the rumen and enter the small and large intestines has limited the interest in intestinal functional probiotics in ruminants.

The rumen acts as a major barrier to bacterial passage in ruminants and experiments have suggested that less than 10% of a bacterial culture added to the diet can be recovered leaving the rumen. Engulfment and digestion of bacteria by protozoa is responsible for the majority of bacterial breakdown in the rumen. The first and limiting step in bacterial breakdown by rumen protozoa is the degradation of the bacterial cell wall. Previous studies have shown that this breakdown is strongly effected by the composition and make up of the bacterial cell wall and that indeed by growing bacteria in the presence of a continuous stress from the cell wall degrading enzyme lysozyme it is possible to "harden" the bacterial cell wall making it more resistant to protozoal predation.

In ruminants, ingested feed enters into the reticulo-rumen, the first of the multiple stomach compartments possessed by ruminants. Within the reticulo-rumen, the ingested feed is pre-digested or degraded by microbial fermentation. Considerable amounts of ingested protein are degraded in the reticulo-rumen to soluble peptides and amino acids. A proportion of these peptides and amino acids are wastefully converted to ammonia and no longer of use to the ruminant. The remainder is utilized by the rumen micro-organisms and incorporated into their own biomass. When the rumen contents pass into the abomasum and intestine, a proportion of the rumen microbial biomass passes out of the reticulo-rumen with the rest of the rumen contents. This microbial biomass is subsequently digested in the small intestine, providing nutrients to the ruminant. However, a significant proportion of the bacteria present within the reticulo-rumen are consumed and digested by the resident protozoal population within the reticulo-rumen. This is a wasteful process for the host ruminant because the bacterial cells and the nutrients contained within the cells do not pass out of the rumen and do not contribute to the nutrition of the ruminant.

In a similar manner, animals are fed bacterial preparations that will adhere to intestinal epithelium thus improving animal growth rate and feed conversion. (U.S. Pat. No. 4,980,164, U.S. Pat. No. 5,256,425). However, in ruminants, the bacterial preparations also have a low survival rate when passing through the rumen. To overcome the loss in viability with oral administration, Batich (U.S. Pat. No. 6,242,230) describes a process for encapsulating bacteria within a gel matrix so they can be delivered to the small intestine of animals. The purpose of Batich is to prevent the host animal from generating an immunological response toward the bacteria, thereby reducing their survivability. Batich is only designed to overcome host immunological response and does not convey any resistance to protozoal digestion and thus the hydrolytic conditions of the rumen can result in degradation of the encapsulating matrix. This is also a costly process and uses chemicals that can reduce the viability of certain microorganisms.

Because yeasts are many-fold larger than bacteria, they are not susceptible to protozoal predation within the rumen as are bacteria but are typically susceptible to lysis within the rumen. Shiozaki et. al. (U.S. Patent No. 4,562,149) describe a method of growing a yeast, *Saccharomyces cerevisiae,* in such a way that the cell is enriched to between 10 and 20% S-adenosyl methionine. This invention is an attempt to use yeast, rather than bacteria, to synthesize methionine. While novel, it is not economically feasible as yeasts are less efficient than bacteria for synthesizing such amino acids. Additionally, no evidence is provided to indicate that this method produces a product that is resistant to degradation of the methionine within the rumen.

Similarly, Ohsumi et al., Biosci. Biotech. Biochem. 58, 1302-1305 (1994) describe a method of growing yeast that is enriched for lysine content. While the lysine content was increased above what is normally observed in wild type yeast, the process has not been deemed economical as a method of producing rumen bypass lysine. Strauss et al., Can. J. Anim. Sci. (2004), used *Pichia pastoris* another species of yeast to demonstrate that when this organism is genetically engineered, it can be used to deliver certain recombinant proteins to the small intestine of ruminants. However, Pichia *pastoris* is not considered as safe to feed to livestock.

Bolla et al (US Pat. No. 6,737,262) describes a method of incorporate-ing fungi or other microorganisms into feed whereby the organism has been genetically transformed to produce peptides of at least two amino acids, rather than individual amino acids. Additionally, the inventors state that further encapsulation may be needed to ensure that the peptides bypass the rumen environment.

In all of the above cases, the manipulation of the yeast cells, either by intensive selection or genetic manipulation is required. Typically *Saccharomyces cerevisiae* are fed to livestock to provide rumen available nutrients and are not particularly well suited for producing large quantities of compounds that would be bioactive in the small intestine. While bacteria can be used commercially to produce a wider range of biologically active compounds and nutrients than yeast, the goal is to have the compounds excreted out of the cell to make the compounds easier to isolate. There is no known method invented whereby bacterial reparations are grown, whether by intensive strain selection or via culturable conditions, so the bacteria and the bioactive compounds contained within, are protected from ruminal degradation.

In producing bacterial preparations, nutrients and other compounds that have bioactive properties, intended for administration to ruminants, it is important to protect the active ingredients against the microbial degradation that occurs within the rumen. It is well known that the rate of meat, wool and/or milk production can be Increased if sources of growth limiting essential amino acids and other bioactive compounds are protected from alteration by rumen microorganisms and are subsequently available for absorption by the animal later in the gastrointestinal tract.

Numerous inventions exist to make biologically active compounds and nutrients stable within the rumen by encapsulation with a coating or by em-bedding the compound within a chemical matrix. U.S. Patent No. 3,959,493, teaches rumen-stable products comprising biologically active substances protected with aliphatic fatty acids. US Pat. No. 3,655,864, issued to Grass et al., teaches veterinary compositions permitting post ruminal delivery of biologically active feed additives, in which the compositions are embedded in or coated within a matrix of glyceryl tristearate with a liquid unsaturated higher fatty acid.

US Pat. No. 4,473,545, issued to Drake et al., teaches an animal feed additive comprising a composite of a relatively insoluble binder, a particulate soluble material and an active material. The particulate material is such that it is readily soluble under a particular range of pH conditions. Dissolution of the particulate materials renders the binder water permeable thus releasing the active material.

U.S. Patent No. 4,533,557 teaches a feed additive for ruminants comprising a mixture in tablet or granule form of at least one biologically active ingredient, chitosan and a protective material of long chain fatty acids. U.S. Patent No. 6,238,727 and U.S. Patent No. 5,885,610 describes the manufacture of insoluble mineral salts of essential amino acids so that they are insoluble in the rumen and thus unavailable for microbial degradation but subsequently available for absorption in the small intestine.

Klose (U.S. Patent No. 6,013,286) describes a composition of matter and method for administering a bioactive compound to ruminants so that the compound does not enter the rumen directly but is passed to the small intestine intact. This method requires that the material have a specific gravity between about 0.3 and 2.0 and that the particles comprises a core of bioactive substance with a hydrophobic coating completely encapsulating the core. Further, a surfactant is applied to the surface of the hydrophobic coating to ensure that particles do not float on the rumen.

In all of the inventions where bioactive compounds are encapsulated or embedded within matrices designed to protect them form ruminal degrada-tion, it requires the compound first be produced by microbial fermentation or chemical synthesis, then purified and subjected to the encapsulation process. This multi-step process is a costly and inefficient method of producing ruminally protected bioactive compounds. At each step, there is a loss of product and loss of bioactivity within the recovered.

L-Lysine is produced by fermentation with L-lysine-producing strains *Corynebacterium glutamicum*. The productivity of *C. glutamicum* can be improved by strain selection, improvements in fermentation technology (i.e. stirring, oxygen supply, composition of the nutrient media). As well, methods of recombinant DNA technology have been used to improve L-lysine production in strains of *C. glutamicum* by amplifying individual biosynthesis genes. In this manner, increased L-lysine production has been obtained by amplification of a DNA fragment conferring resistance to aminoethylcysteine (EP 88166), feedback-resistant aspartate kinase.(EP 387 527), amplification of dihydrodipicolinate synthase (EP 197 335), aspartate aminotransferase (EP 219 027), phosphoenolpyruvate carboxylase aspartate (EP 143 195 and EP 358 940), semialdehyde dehydrogenase (EP 219 027) and pyruvate carboxyase (DE 198 31 609).

In industrial production of L-lysine, it is necessary to separate the L-lysine product from the bacterial cell to enhance efficiency L-lysine synthesis by the bacteria. It has been discovered that the gene LysE is responsible for exporting L-lysine out of the cytoplasm of *C. glutamicum* and into the media and is critical for efficient industrial L-lysine production (Tryfona et al., Process Biochem (2004)). Increased activity of the LysE L-lysine export carrier promotes lysine production (DE 195 48 222).

The problem that exists is that there is no means of protecting bacteria and other microorganisms from rumen degradation so that they can bypass the rumen and be delivered intact to the small intestine. Likewise the bio-active compounds they produce must be excreted from the bacterial cells so they can be purified. Once purified, the bioactive compounds must be protect-ed against rumen degradation by encapsulation or embedding technology.

SUMMARY OF THE INVENTION

Methods have now been discovered for identifying strains of Gram positive bacteria useful for gastrointestinal delivery of bioactive compounds to ruminants that are resistant to inactivation in the rumen. Other methods have been discovered for increasing resistance to rumen inactivation of cultured bacteria strains useful for gastrointestinal delivery of bioactive compounds to ruminants, regardless of how inherently resistant the bacteria strain may be to rumen inactivation.

Therefore, according to one aspect of the present invention, an in vitro method is provided for evaluating the resistance of a bacteria strain to rumen inactivation in vivo, wherein the method comprises:

culturing in vitro, a Gram positive bacteria strain useful for the gastrointestinal delivery of a bioactive compound to ruminants in a nutrient medium containing natural or synthetic ruminal fluid; and

measuring the protein degradation in the bacteria culture as a function of time.

The ruminal fluid is selected to approximate rumen conditions to be encountered by the bacteria strain to be administered. Natural ruminal fluid is taken from the rumen contents of a healthy ruminant within twenty four hours after feeding. Synthetic ruminal fluid is a mixture of materials selected to simulate conditions in the rumen, including one or more species of predatory protozoa that consume microorganisms in the rumen. Such protozoa species are readily identified by one of ordinary skill in the art.

Preferred methods according to the present invention assay the release of $C^{14}$ labelled leucine to measure protein degradation according to the method of Wallace et al., Br. J. Nutr., 58, 313 - 323 (1987), the disclosure of which is incorporated herein by reference. The results are expressed as a rate described as % of remaining bacteria present that are degraded per hour. For purposes of the present invention, bacteria strains with a degradation rate of less than 8 % per hour are defined as resistant to rumen inactivation. Strains having a degradation rate less than 6 % per hour are preferred for bioactive compound delivery to ruminants, with strains having a degradation rate less than 4 % per hour being more preferred.

Correspondingly, strains that are resistant to rumen inactivation will have more than 20% of the dosage of bacteria fed to an animal per day delivered through the reticulo-rumen intact. Preferred strains will have more than 50% of the dosage of bacteria fed to an animal per day delivered through the reticulo-rumen intact and more preferred will have more than 80% of the dosage of bacteria fed to an animal per day delivered through the reticulo-rumen intact

Accordingly, one embodiment of this aspect of the invention further includes the step of identifying as resistant to rumen inactivation bacterial strains having a degradation rate of less than 8 % per hour as measured by the release of $C^{14}$ labelled leucine according to the method of Wallace et al.

According to another embodiment of this aspect of the invention the useful bacteria strain is a lysine-producing bacteria strain, preferably a strain of Comyebacterium glutamicum, and more preferably a C. glutamicum strain known for overproduction of lysine, including C. glutamicum strains genetic-ally modified to overproduce lysine. However, this method may be applied to essentially any bacteria species that is useful for the gastrointestinal delivery of a bioactive compound to a ruminant for which an evaluation of resistance to rumen inactivation is desired.

For purposes of the present invention, "gastrointestinal delivery" is defined as including delivery to the abomasum, small intestine and large intestine of a ruminant. Exactly where the bioactive compound is delivered depends upon the nature of the bioactive compound to be delivered, which is understood by one of ordinary skill in the art seeking to administer the compound. The present invention does not modify the location of delivery but protects the bioactive compound from rumen inactivation as it is being delivered.

The method according to this aspect of the invention provides the ability to select bacterial strains with reduced rumen degradability that can be used to deliver gastrointestinally specific bacteria, and bioactive compounds contained within them to a ruminant, wherein the bacteria cell wall serves to provide rumen bypass protection to the cell contents. The bacteria strains selected may have adequate resistance to rumen degradation to permit feeding of the useful bacteria biomass to ruminants without further modification.

Strains that have been discovered to have adequate resistance to rumen modification to permit feeding of the cell contents to ruminants without further modification include C. glutamicum ATCC strains 13058, 13825, 14066, 14067, 14068, 21127 and 700239, Therefore, according to another aspect of the present invention, a rumen bypass feed supplement is provided containing the lysine-containing biomass of a C. glutamicum strain selected from the group

consisting of <u>C. glutamicum</u> ATCC strains 13058, 13825, 14066, 14067, 14068, 21127 and 700239.

The present invention also provides a method by which bacteria strains may be rendered more resistant to rumen inactivation. The method according to this aspect of the invention can be used to increase resistance to rumen inactivation of bacteria strains identified as rumen inactivation resistant and those that are not.

Therefore, according to another aspect of the invention, a method is provided for increasing the resistance of a cultured bacteria strain to rumen inactivation, wherein the bacteria strain is a gram positive bacteria strain that is nutritionally beneficial to ruminants, and the method includes the steps of:

growing a culture of the bacterial strain through at least one passage in a growth medium containing an amount of lysozyme effective to induce the growth of bacterial cell walls resistant to protozoal predation; and

recovering the bacterial strain from the lysozyme-containing medium.

According to one embodiment of this aspect of the invention, the concentration of the lysozyme in the growth medium is between about 1 and about 100 ug/ml. According to another embodiment of this aspect of the invention a plurality of growth passages are used, with the preferred number of passages being between about 2 and about 20.

According to yet another embodiment of this aspect of the invention, the recovering step is performed after the last passage after which the bacterial biomass is recovered in which the bacteria cell walls, which are resistant to rumen degradation. The biomass is then preferably de-watered and concentrated for feeding to a ruminant by conventional means.

In another embodiment of this aspect of the invention the bacteria strain is a lysine-producing bacteria strain, preferably a strain of <u>Cornye-bacterium glutamicum</u>, and more preferably a <u>C. glutamicum</u> strain known for overproduction of lysine, including strains genetically modified to overproduce lysine. However, this method may likewise be applied to essentially any bacteria species useful for gastrointestinal delivery of bioactive compounds to ruminants for which an increase in resistance to rumen inactivation is desired.

The present invention also includes rumen bypass feed supplements containing bacteria biomass useful for gastrointestinal delivery of bioactive compounds to ruminants that are resistant to rumen inactivation obtained by either method according to the present invention and methods for supplernen-ting the diet of a ruminant with the rumen bypass feed supplements. When included in animal feed and offered to ruminants, the bacteria function as a system for gastrointestinally delivering bioactive compounds to ruminants.

The foregoing and other objects, features and advantages of the present invention are more readily apparent from the detailed description of the preferred embodiments set forth below, taken in conjunction with the accompanying drawings.

BRIEF DESCRIPTION OF THE DRAWINGS

FIG. 1 depicts the degradation rate of two strains of *C. glutamicum* not grown in the presence of lysozyme compared to *S. ruminantium* Z108;

FIG. 2 depicts the degradation rate of the same two strains of *C. glutamicum* grown in the presence of lysozyme compared to *S. ruminantium* Z108;

FIG. 3 depicts the amount of breakdown in rumen fluid of *C. gluta-micum* strains ATCC 13869, 700239 and 31269 grown in the presence and absence of lysozyme compared to *S. bovis* ES1;

FIG. 4 depicts the rate of breakdown in rumen fluid for the same *C. glutamicum* strains grown in the presence and absence of lysozyme compared to *S. bovis* ES1; and

FIG. 5 depicts the breakdown in rumen fluid from cattle of *Bifidobacter. longum, Propionibacterium freudenreichii , Lactobacillus raffinolactis, Lacto. fermentum Lactobacillus lactis, Lactobacillus pentosus* and *Propionibacter. acidipropionici* grown In the presence and absence of lysozyme compared to *S. bovis* ES1.

DETAILED DESCRIPTION OF PREFERRED EMBODIMENTS

To impart resistance to rumen degradation, useful bacteria are grown in nutrient media in the presence of lysozyme, preferably under fermentation conditions that are ideal for the growth of the specific organism in commercial quantities and optimized for synthesis of the bioactive compound of interest. Examples of suitable nutrient media include Lennox Medium (Kumagai et. al. Bioscience, Biotechnology, and Biochemistry, 69, 2051-2056 (2005)), CGXII Medium (Keilhauer et al. 1993. J Bacteriol 175: 5595-5603), Luria Bertani Broth (Lennox, E. S. 1955. Virology 1:190-206) and the complex media described by Broer & Kramer (J. Bacteriol. 1990, 172, 7241-7248).

Lysozyme is added to the nutrient medium at a concentration effective to strengthen resistance to lysing in the rumen. The lysozyme concentration should not be so low that no statistical or commercially significant improve-ment in rumen degradation performance is observed, or so high that cell growth is unacceptably inhibited. Accordingly, the lysozyme concentration is preferably between about 0.1 and about 100 ug/ml, and more preferably between about 1 and about 10 ug/ml.

Preferred methods employ a plurality of serial passages in lysozyme-containing growth medium. Methods employing between about 2 and about 10 serial passages are more preferred. The bacteria are grown in each passage for between about 12 and about 48 hours with a total growth time in the presence of lysozyme between about 1 and about 20 days preferred.

The bacteria cells are then harvested by filtration and/or centrifugation, concentrated and/or dried and packaged in a commercially acceptable manner.

The method of the present invention that employs lysozyme to impart resistance to rumen inactivation can be applied to any bacteria species useful for gastrointestinal delivery of bioactive compounds to ruminants. Examples of such species include, but are not limited to, *Bifidobacterium infantis, Lacto-bacillus reuteri, Bifidobacferium longum, Leuconostoc mesenteroides, Bacillus coagulans, Bifidobacterium thermophilum, Pediococcus acidilactici, Bacillus lentus, Lactobacillus acidophilus, Pediococc. cerevis. (damnosus), Bacillus licheniformis, Lactobacillus brevis, Pediococcus pentosaceus, Bacillus pumi-lus, Lactobacillus bulgaricus, Propionibacter. freudenreichii, Bacillus subtilis, Lactobacillus casei, Propionibacterium shermanii, Bacteroides amylophilus, Lactobacillus cellobiosus, Bacteroides capillosus, Lactobacillus curvatus, Streptococcus cremoirs, Bacteriodes ruminicola, Lactobacillus delbrueckii, Streptococcus diacetilactis, Bacteroides suis, Lactobacillus fermentum, Streptococcus faecium, Bifidobacterium adolescentis, Lactobacillus helveti-cus, Streptococcus interimedius, Bifidobacterium animatis, Lactobacillus lactis, Streptococcus lactis, Bifidobacterium bifidum, Lactobacillus plantarum* and *Streptococcus thermophilus.*

The resulting feed additives also confer useful benefits to monogastric animals, including humans, even though rumen-bypass properties are not required.

The invention is particularly well-suited for use with Gram positive bacteria because of their thick peptidoglycan cell wall. Examples of Gram positive bacteria include mycobacteria, nocardia, lactobacillus, streptococcus, *Bacillus* and <u>Corynebacteria</u>. Many commercially useful lysine-producing strains of *C. glutamicum* have been developed that are well-suited for use with the present invention such as ATCC strains 13058, 13825, 14066, 14067, 14068, 21127 and 700239. *Corynebacteria glutamicum* strains capable of synthesizing high concentrations of L-lysine are preferred such as ATCC strains 21127 and 700239. *Corynebacteria. glutamicum* strains that are deficient in the exporter gene LysE are also preferred.

Bioactive compounds that may be delivered by bacteria species grown in the presence of lysozyme include nutrients such as amino acids, deriva-tives thereof, hydroxy homologues of amino acids, proteins, carbohydrates, fats, vitamins, and animal drugs, alone or as a mixture of two or more.

Illustrative examples of bioactive compounds include amino acids such as lysine, methionine, tryptophan, threonine, etc.; amino acid derivatives such as N-acylamino acids, N-hydroxymethylmethionine calcium salt, lysine HCl, etc.; amino acid hydroxy homologues such as 2-hydroxy-4-methylmercapto-butyric acid and salts thereof, etc.; carbohydrates such as starch, sucrose, glucose, etc.; fats such as polyunsaturated fatty acids, omega-3 fatty acids, omega-6 fatty acids, trans fatty acids, etc.; and vitamins and substances with a function similar to vitamins such as vitamin A, vitamin A acetate, vitamin A palmitate, B vitamins such as thiamine, thiamine HCl, riboflavin, nicotinic acid, nicotinamide, calcium pantothenate, choline pantothenate, pyridoxine HCl, choline chloride, cyanocobalamin, biotin, folic acid, etc., p-aminobenzoic acid, vitamins D2 and D3, vitamin E, etc.

In addition to nutrients, bioactive compounds also include therapeutic compounds including hormones such as estrogen, stilbestrol, hexestrol, thyro-protein, goitrogen, growth hormone, etc. Bioactive therapeutic com-pounds also include therapeutic peptides and proteins, including enzymes such as amylase, protease, xylanase, pectinase, cellulase, lactase, lipase, etc.; hormonal proteins such as growth hormone, somatotropin, etc.; microbial binding carbohydrates such as mannan- and fructo-oligosaccharides and anti-microbial peptide compounds such as bacteriocins.

Accordingly, the method of the present invention, in addition to being useful with both naturally occurring bacterial strains and strains produced by intensive selection processes, can also be applied to recombinantly produced bacteria strains. A recombinant bacteria strain genetically engineered to produce a desired therapeutic peptide or protein can then be modified by the inventive method to enable the recombinant bacteria cells to safely bypass the rumen for gastrointestinal delivery of the peptide or protein.

The bacteria cells themselves can also have value for gastrointestinal delivery of compounds contained on the cell surface of the bacteria. In addition, cells with no nutritional value that function to compete with patho-gens in the intestine (i.e., competitive exclusion) are also included, within the scope of the definition of "bioactive compounds" for purposes of the present invention.

A separate <u>in vitro</u> method is also provided to identify useful bacteria strains that are either inert to rumen inactivation or must be grown in a lysozyme-containing growth medium to be rendered inert to rumen inactivation. This method of evaluating useful bacteria strains for rumen inactivation resistance may be applied to the above-identified useful bacteria species. The method serves to identify strains that are inherently resistant to rumen inactivation and have utility as rumen bypass feed supplements without first being grown in a lysozyme-containing media and strains to which resistance to rumen inactivation must first be imparted by culturing in the presence of lysozyme.

The <u>in vitro</u> method cultivates a Gram positive bacteria strain useful for the gastrointestinal delivery of a bioactive compound to ruminants in a growth medium containing natural or synthetic ruminal fluid and measures protein degradation as a function of time. The growth medium will contain from about 80 and about 99 % by volume of a nutrient media and from about 1 and about 20 % by volume of ruminal fluid. Examples of suitable nutrient media include Dehority's Medium (Scott and Dehority, J. Bacteriol., 89, 1169-1175 (1965)), Hobson's M2 Medium (Hobson, Methods Microbiol., 3B, 133-149 (1969)) and CRT Medium (Wallace et. al., Int. J. Syst. Evol. Microbiol., 53, 965-970 (2003)). Numerous other suitable media are described in the books by Hungate (Hungate R E 1966. The rumen and its microbes. Academic Press, New York, NY) and Hobson & Stewart (The Rumen Microbial Ecosystem, Chapman and Hall, London).

The ruminal fluid is selected to approximate rumen conditions. Natural ruminal fluid is obtained from the rumen contents of healthy ruminants. For example, the fluid may be withdrawn from rumen-fistulated: ruminants. The fluid is preferably obtained from the same ruminant species, and preferably from ruminants subject to the same feeding conditions as the ruminants to which the bacteria strain will be administered. The fluid is preferably obtained within 24 hours after feeding, and more preferably within about one to about three hours after the morning feeding. The ruminal fluid should be strained to remove unwanted particulate matter.

Synthetic ruminal fluid is prepared from materials that simulate the conditions to be encountered in the rumen. The fluid will contain one or more species of predatory protozoa that consume microorganisms in the rumen and the nutrients for growth of the bacteria which include sugars, phosphate and bicarbonate buffers, mineral salts, volatile fatty acids and vitamins. Examples of synthetic media are well described by Hobson & Stewart (The Rumen Microbial Ecosystem, Chapman and Hall, London). Examples of ruminal protozoa responsible for bacterial degradation include *Epidinium, Eudiplodinium, Isotricha Dasytricha, Entodinium* and *Polyplastron* species (Ivan et. al. 2000aJ Anim Sci 78, 750-759; lvan et. al. 2000b. J Dairy Sci 83, 776-787).

The useful bacteria strain to be evaluated is cultivated in the growth media under the temperature conditions to be encountered in the rumen, i.e., between about 36 and about 40 C. The incubation time may be selected to approximate the amount of time the bacteria strain will reside in the rumen, typically between about one and about 48 hours. A longer time can be used to obtain a greater amount of protein degradation data, for example, from 12 to about 48 hours. The amount of protein degradation expected for the amount of time the bacteria strain will actually spend in the rumen can then be extrapolated from this data.

Protein degradation for the bacteria strain is measured in terms of the weight of degradation product or products produced as a function of time. One preferred method according to the present invention assays the release of $C^{14}$ labelled leucine to measure protein degradation according to the method of Wallace et al., Br. J. Nutr., 58, 313 - 323 (1987), the disclosure of which is incorporated herein by reference. The results are expressed as a rate described as % of remaining bacteria present that are degraded per hour. For purposes of the present invention, bacteria.strains with at degradation rate of less than 8 % per hour are defined as resistant to rumen inactivation. Strains having a degradation rate less than 6% per hour are preferred for bioactive compound delivery to ruminants, with strains having a degradation rate less than 4 % per hour being more preferred.

Bacteria strains that following evaluation are not considered resistant to rumen degradation, i.e., strains that have a degradation rate greater than 8% per hour, may then be grown in the presence of lysozyme to improve resistance to rumen degradation. The improvement may be measured by re-evaluating the bacteria strain after lysozyme exposure with the in vitro evaluation method of the present invention using natural or synthetic ruminal fluid. The degree of improvement can be expressed as the percent reduction in the degradation rate over the same unit of time following lysozyme exposure. However, the degree of improvement is not as important as having the rate of degradation fall below the threshold required for the bacteria strain to be con-sidered resistant to rumen degradation as defined by the present application. That is, a large increase in resistance may still be insufficient while a small increase may be more than adequate.

Useful bacteria strains identified as resistant to rumen degradation, or resistant to rumen degradation when grown in the presence of lysozyme, may then be grown (with lysozyme if necessary for rumen degradation resistance) and biomass harvested in commercial quantities with commercial fermentation equipment including batch, fed-batch and continuous culture equipment The biomass may then be optionally blended with acceptable fillers, binders, flavor additives, and the like, to form a rumen bypass feed supplement, or the bio-mass itself may serve as the feed supplement to be admixed with a ruminant feed ration. Alternatively, the biomass and other additives may be dissolved or suspended in an aqueous medium to form a rumen bypass feed supplement that is sprayed onto the feed ration. The formation of either dosage form is essentially conventional and well-known to one of ordinary skill in the art. Other known ruminant nutritional ingredients may be added to either form of rumen bypass feed supplement.

The harvested bacteria cells resistant to rumen degradation may be conveniently fed to a ruminant admixed with a conventional ruminant feed. The feeds are typically vegetable materials edible by ruminants, such as legume hay, grass hay, com silage, grass silage, legume silage, corn grain, oats, barley, distiller's grain, brewer's grain, soy bean meal and cottonseed meal and are included in an amount as typically recommended by a husbandry nutritionist, which ordinarily does not exceed 5% by weight of the dry solids content of the feed.

For a rumen-protected lysine feed supplement, such as a feed supplement containing C. glutamicum biomass, the amount of supplement to be added to the dry solids content of the feed should be an amount effective to supply a daily average of between about 5 and about 150 mg of metabolically available lysine per kg of ruminant body weight. An amount of metabolically available lysine between about 15 and about 75 g per kg of ruminant body weight is preferred. Metabolically available lysine can be measured by determining the flow of lysine from an in vitro rumen simulation system; measuring the flow of lysine to the small intestine in animals fitted with abomasal and/or intestinal cannulae or by measuring the increase in milk protein percentage and/or yield in female ruminants fed a diet designed to be deficient in metabolically available lysine. There are numerous permutations of these methods known to those ordinary in the art that can be used to determine metabolically available lysine.

The rumen bypass feed supplements of the present invention may be fed to any ruminant in need of nutritional supplementation, including livestock, research animals and animals on display in zoos and other wildlife exhibits. Examples of ruminants include cattle, oxen, sheep and goats. The rumen bypass feed supplements can be fed to livestock raised for meat, milk, hide, hair or wool, or ruminants used as work animals on a farm.

The following non-limiting examples set forth herein below illustrate certain aspects of the invention. All parts and percentages are by weight unless otherwise noted, and all temperatures are in degrees Celsius.

EXAMPLES

Example 1. Susceptibility of C. glutamicum strains to ruminal degradation via protozoal predation.

The degradation of C. glutamicum and S. ruminantium (representing an "average" rumen bacteria) was determined in rumen fluid according to the method described by Wallace et al., Br. J. Nutr., 58, 313-323 (1987).

Corynebacteria glutamicum strains (ATCC 13761 and ATCC 13869 were grown in aerobic Wallace and McPherson medium. S. ruminantium Z108 was grown in anaerobic Wallace and McPherson medium. The Wallace and McPherson media and the preparation thereof are disclosed by the above-refereced Wallace et al. journal article. Cultures were grown overnight at 39 C. Cells were harvested by centrifuging at 1000 g x 10 min at room temperature. Cells were resuspended in anaerobic Cole-man's buffer containing 5 mM $C^{14}$ L-leucine and incubated overnight (OD = 1.0) to label bacterial protein. A sample (1 ml) was removed and placed into 0.25 ml 25% TCA for protein determination. Two 50 $\mu$l aliquots were placed into scintiiia-tion fluid to determine amount of radioactivity added.

Rumen fluid was removed from three sheep 2 hr after feeding and strained through muslin. Unlabelled L-leucine (5 mM) was added and strained rumen fluid (SRF) was kept warm. A sample (I mL) was added to 1 mL of 4% formalin for protozoa counts.

Labelled bacterial cell suspension (0.5 ml) was added to 4.5 ml of SRF or buffer and incubated at 39°C. Samples (0.5 ml) were removed at 0, 1, 2 and 3 hrs and placed into into 0.125 ml TCA. Samples were centrifuged at 14 000 rpm for 3 min and 200 $\mu$l supernatant was counted to determine released radioactivity, representing bacterial protein degraded by protozoa.

Based on the release of radioactivity, representing bacterial protein degraded by protozoa, percent degradation was determined at each time point. Data was fitted to the equation (Mehrez and Orskov, 1987):

$$Y = a + (c - a) * 1\text{-}(\exp[-k_d*x]); \text{ where } Y = \text{degradation at a specified time } x, \text{ hr;}$$

a = initial degradation; c = maximum degradation; $k_d$ = rate of degradation, $hr^{-1}$;

Effective degradability was determined for selected ruminal rates of passage according to the equation:

$$Y = a + (c * k_d)/(K_d + K_p) \text{ where } K_p = \text{ruminal turnover rate.}$$

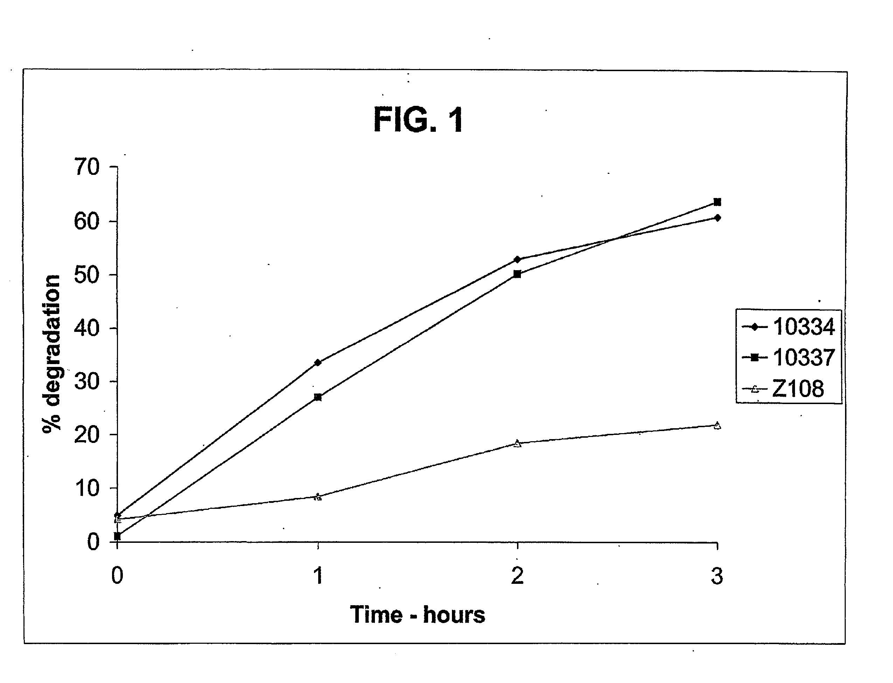

The results are shown in FIG. 1. Both strains of C. glutamicum showed rapid degradation compared to S. ruminantium Z108. Disappearance over the 3 hr. incubation period was 71.2 and 83.1 % for strains 10336 and 13869 respectively compared to 21.9 % for S. ruminantium Z108. Effective degradability at a rumen turnover rate of 0.07 $hr^{-1}$ was 71.2 and 53.8 % for strains 13761 and 13869 respectively.

Example 2. C. glutamicum growth in the presence of low lysozyme levels

Wallace and McPherson non $C^{14}$ media was prepared (24 x 7 ml). To each of 4 sets of tubes, 0.5 ml of filter sterilized lysozyme (0.1; 1.0; 10; 100; or 1000 μg/ml) was added. To a final set of 4 tubes, 0.5 ml of Coleman's D media was added (Control). Tubes were inoculated with cultures of *C. gluta-micum* strain ATCC 13761 and incubated at 39°C for 48 hr and OD (650 nm) was measured for each organism at 24 and 48 hr.

Strain 13761 grew well at the lowest two levels of lysozyme exposure (0.1 and 1 μg/ml). Growth was cut in half with 100 μg/ml and completely inhibited at 1000 μg/ml.

Example 3. Effect of growth of *C. glutamicum* strains in the presence of lysozyme on susceptibility to protozoal predation

Methodology was essentially the same as Experiment 1, except that treatments consisted of *C. glutamicum* strains ATCC 13761 and ATCC 13869 grown as in Experiment 1 or in the presence of lysozyme (0.5 ml of 0.25 μg/ml lysozyme; 16.7 μg/ml final concentration). As in Experiment 1; *S. ruminantium* Z108 was used as a check organism.

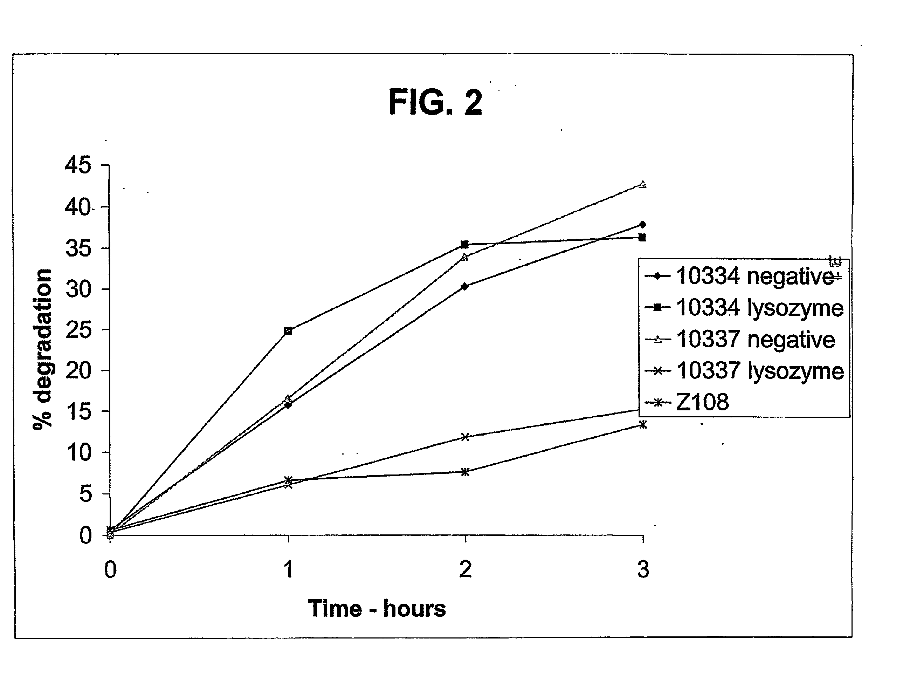

The results are shown in FIG. 2. Degradation was lower in this experiment than in Experiment 1 for all organisms. When strains were grown without lysozyme (native) disappearance over the 3 hr. incubation period was 37.9 and 42.8 % for strains 13761 and 13869 respectively compared to 13.3 % for *S. ruminantium* Z108. However, when grown in the presence of lysozyme, 3 hr degradation was reduced to 15.2 % for strain 13869 but unaffected for strain 13761 (36.3 %). Effective degradability at a rumen turnover rate of 0.07 hr$^{-1}$ was 53.8 and 64.7 % for strains 13761 and 13869 respectively when not grown in the presence of lysozyme and 36.1 and 24.5 % for the respective strains when grown in the presence of lysozyme.

Examples 4-6. Effect of growth of *C. glutamicum* strains 13869, 700239 and 31269 in the presence of lysozyme on susceptibility to protozoal predation

*C. glutamicum* strains 13869, 700239 and 31269 were obtained from the American Type Culture Collection (ATCC). Cultures were revived on nutrient agar and transferred to nutrient broth as per ATCC instructions. When healthy growth was observed (after 3 x 24 h passages through nutrient broth at 39 C) cultures were grown for a further 3 x 24h at 39 C in nutrient broth plus or minus 20 μg/ml hens egg white lysozyme. *S. bovis* ES1 was previously isolated from the rumen of a sheep and is maintained within the culture collection of the Institute of Rural Sciences, University of Wales, Aberystwyth.

Bacteria were labelled by growing cultures for 24 h at 39 C in a rumen fluid-containing Wallace and McPherson medium with ammonia cysteine and L-[U-$^{14}$C]leucine as the only added N sources, and with 20 μg/ml hens egg white lysozyme was added to cultures previously grown in the presence of lysozyme. Cells were harvested by centrifugation and washed once in Coleman's salts solution D (Coleman, 1978) before being incubated with strained ruminal fluid. Ruminal fluid was withdrawn 2 h after the morning feeding from 3 rumen-fistulated cattle receiving a grass silage based ration. The fluid was strained through 4 layers of muslin cloth.

Apparent protein degradation was measured by the release of [$^{14}$C] into trichloroacetic acid-soluble material during 3-h incubations. Unlabelled L-leucine was included in all incubations at a final concentration of 5 mmol/ L.

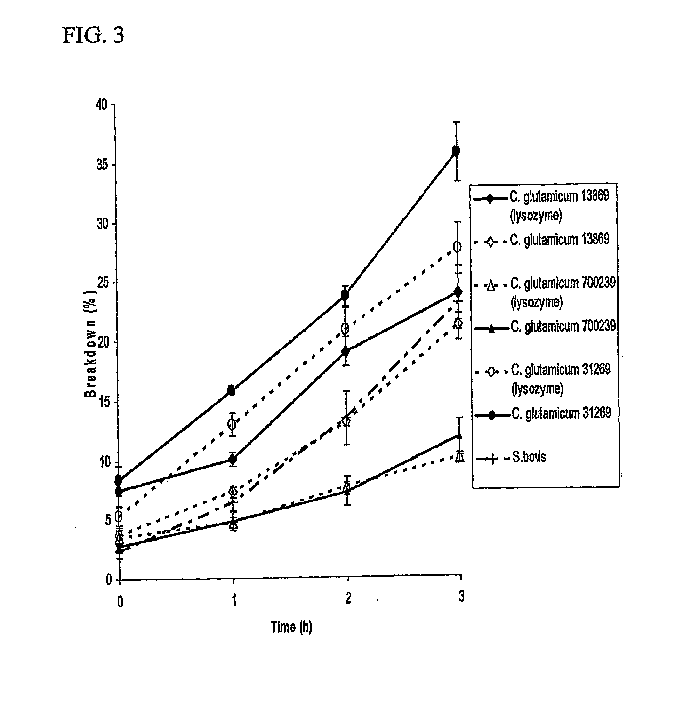

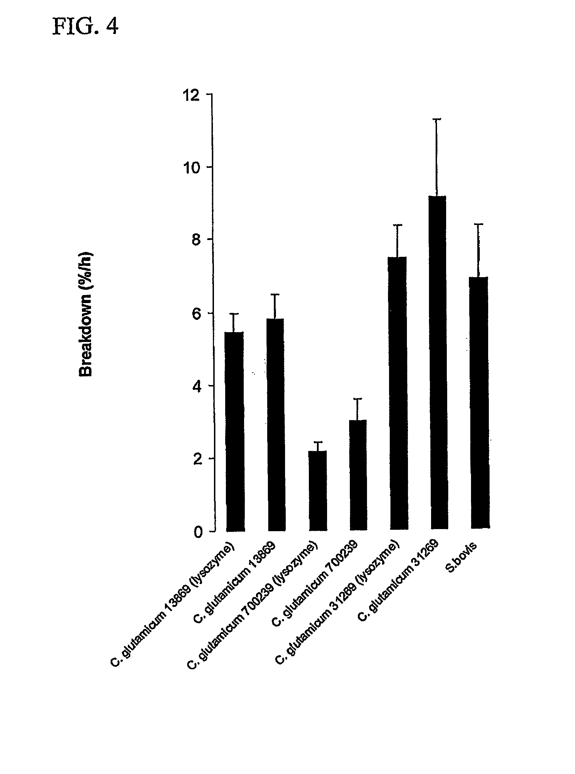

The breakdown of the different bacteria over the three hours incubation in rumen fluid is shown in Fig. 3. An initial comparison of the rate of bacteria breakdown (as %/h) showed that *C. glutamicum* strain 700239 was broken down significantly slower than either of the other *C. glutamicum* strains or the rumen bacterium S. bovis (5.63, 2.58, 8.27, 6.88 %/h SED 1.287 for *C. glutamicum* strains 13869, 700239 and 31269 and S. bovis ES1 respectively, Figure 4). When the data for the *C. glutamicum* strains alone was examined it was clear that while again there was a significant difference in the rate of breakdown between the strains used there was no improvement for these strains following pre-incubation with lysozyme (Table 1).

Table 1 - Effect of growth in the presence and absence of lysozyme on the breakdown of *C. glutamicum* strains 13869, 700239 and 31269 in rumen fluid

|  | Grown in the presence of lysozyme | Grown in the absence of lysozyme |
|---|---|---|
|  | Breakdown (%/h) |  |
| *C. glutamicum* |  |  |
| 13869 | 5.5 | 5.8 |
| 700239 | 2.2 | 3.0 |
| 31269 | 7.4 | 9.1 |
| SED |  |  |
| Strain | 1.03[***] |  |

(continued)

|  | Grown in the presence of lysozyme | Grown in the absence of lysozyme |
|---|---|---|
| Lysozyme | 0.84[ns] | |
| Strain x lysozyme interaction | 1.46[hs] | |

The assay used here was based on that described by Wallace et al., wherein the release of $C^{14}$ leucine from bacteria in rumen fluid in the presence of an excess of unlabelled leucine is used to measure the breakdown of bacteria in the rumen. For the three bacteria strains, growing the cultures in lysozyme had no significant effect on the rate of breakdown in rumen fluid, despite there being a numerical decrease of circa 16%. Two possible reasons for this lack of effect are presented below:

*Time in culture*: in the current experiment *C. glutamicum* was incubated with lysozyme for a total of 96 h (3 passages of 24 through 20 $\mu$g/ml in nutrient broth plus one in the Wallace and McPherson media used to label the cells). It is possible that insufficient time was allowed for the cultures to change their cell structure in response to the lysozyme.

*Low degradability of the cultures even in the absence* of *lysozyme*: in the current experiment the degradability of the *C. glutamicum* strains grown in the absence of lysozyme varied from 9 to 3%/h. This is considerably lower than the values previously recorded with other strains of *C. glutamicum* i.e. (12 and 14%h with strains 10334 and 10337) and very much lower from the figures recorded with *B. fibrisolvens* (circa 30%/h) wherein the initial observations that lysozyme could reduce degradation where made. In contrast the figures for *S. bovis* recorded here are very similar to those observed previously. It is possible that the reason lysozyme conferred little protective effect was that the cells were already relatively resistant to protozoal attack.

It is noteworthy that when *C. glutamicum* 700239 was grown in the presence of lysozyme the rate of degradation was about 2%/ h. Assuming that when added to the rumen *C. glutamicum* 700239 would leave the rumen at a relatively modest 10%/h in the liquid phase, then over a 24h period about 77% of the *C. glutamicum* 700239 would bypass the rumen with less than 15% being degraded. At a more realistic 15%/h liquid turnover over 85% would bypass the rumen with lass than 12% being degraded.

In the current experiment growth in the presence of lysozyme did not apparently protect the strains of *C. glutamicum* investigated against degrada-tion in the rumen. However, *C. glutamicum* strain 700239 is remarkably resistant to breakdown in the rumen is expected to supply a suitable vector to passage amino acids such as lysine through the rumen.

Examples 7-13. Effect of growth of *Bifidobacterium longum, Propionoibacerium freudenreichii, Lactobacillus raffinolactis, Lactobacillus fermentum, Lactobacillus lactis, Lactobacillus pentosus* and *Propionibacerium acidipropionici* strains in the presence of lysozyme on susceptibility to protozoal predation.

The effect on breakdown in rumen fluid of seven different potentially probiotic organisms other than *C. glutamicum,* was investigated by growing the organisms in the presence of lysozyme *in vitro,* using as a control a typical rumen bacterium, *Streptococcus bovis,* as in Examples 1 - 6.

*Bifidobacterium longum, P. freudenreichii* and *P. acidipropionici* were obtained from the National Collection of industrial and Marine Bacteria (NCIMB) and the National Collection of Food Bacteria, Aberdeen. *Lactobacillus raffinolactis, Lactobacillus fermentum, Lactobacillus lactis* and *Lactobacillus pentosus* were obtained from Dr Kevin Hillman, Gutbugs, UK

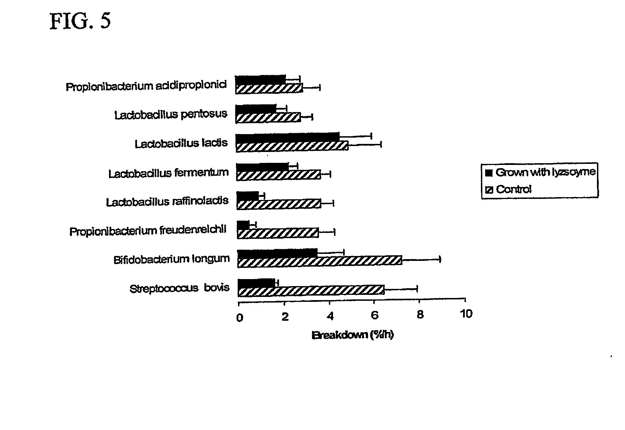

Bacteria were grown and labelled and apparent protein breakdown measured as in Examples 4-6, including *S. bovis* ES1. Breakdown of the different bacteria over three hrs incubation in rumen fluid is shown in Fig. 5. Growth in the presence of lysozyme decreased the breakdown of *S. bovis, P. freuden-reichii* and *L. raffinolactis* by more than 70%. The breakdown of *L. pentosus, B. longum* and *L. fermentum* decreased by between 40 and 50% while there was no effect on the breakdown of L. *lactis* or *P acidipropionici* (Table 2).

Table 2 - Effect of growth in the presence and absence of lysozyme on probiotic organisms in rumen fluid

| | Grown in the absence of lysozyme | Grown in the presence of lysozyme | SED |
|---|---|---|---|
| Breakdown (%/h) | | | |
| Streptococcus bovis | 6.44 | 1.58 | 1.629 |
| Bifidobacterium longum | 7.25 | 3.46 | 0.535 |
| P. freudenreichii | 3.56 | 0.50 | 0.444 |

(continued)

| | Grown in the absence of lysozyme | Grown in the presence of lysozyme | SED |
|---|---|---|---|
| Lactobacillus raffinolactis | 3.66 | 0.92 | 0.307 |
| Lactobacillus fermentum | 3.68 | 2.24 | 0.0702 |
| Lactobacillus lactis | 4.91 | 4.55 | 0.1285NS |
| Lactobacillus pentosus | 2.81 | 1.73 | 0.1317 |
| P. acidipropionici | 2.95 | 2.19 | 0.252NS |

The foregoing examples and description of the preferred embodiments should be taken as illustrating, rather than as limiting, the present invention as defined by the claims. Numerous combinations of the features set forth above can be utilized without departing from the present invention as set forth in the claims. Such variations are not regarded as a departure from the spirit and scope of the invention, and all such modifications are intended to be included within the scope of the following claims.

The following are aspects of the invention:

1. A method for increasing the resistance to rumen inactivation of a lysine-producing Gram positive bacteria strain comprising the steps of:

growing a culture of the bacteria strain through at least one passage in a growth medium containing an amount of lysozyme effective to induce the growth of bacteria cell walls resistant to protozoal predation; and recovering the bacteria strain from the lysozyme-containing medium.

2. The method of aspect 1, wherein said bacteria strain is a strain of *Corynebacteria glutamicum.*

3. The method of aspect 2, wherein said *Corynebacteria glutamicum* strain is an ATCC strain selected from the group consisting 13058, 13825, 14066, 14067, 14068, 21127 and 700239.

4. The method of aspect 2, wherein said *Corynebacteria glutamicum* strain overproduces lysine.

5. The method of aspect 4, wherein said *Corynebacteria glutamicum* strain is genetically modified to overproduce lysine.

6. The method of aspect 1, wherein the lysozyme concentration in said growth medium is between about 0.01 and about 100 ug/ml.

7. The method of aspect 6, wherein said lysozyme concentration is between about 0.1 and about 10 ug/ml.

8. The method of aspect 1, wherein a plurality of passages in lysozyme-containing growth media are employed.

9. The method of aspect 8, where between about 2 and about 10 passages are employed.

10. A rumen-protected lysine feed supplement comprising a lysine-producing bacteria strain grown by the method of aspect 1.

11. The rumen-protected lysine feed supplement of aspect 10, wherein said bacteria strain is a strain of *Corynebacteria glutamicum.*

12. The rumen-protected lysine feed supplement of aspect 11, wherein said *Corynebacteria glutamicum* strain overproduces lysine.

13. The rumen-protected lysine feed supplement of aspect 12, wherein said *Corynebacteria glutamicum* strain is genetically modified to overproduce lysine.

14. The rumen-protected lysine feed supplement of aspect 10, wherein said bacteria strains has a rumen degradation rate of less than about 8% per hour as measured by the release of $C^{14}$ labelled leucine according to the method of Wallace et al.

15. The rumen-protected lysine feed supplement of aspect 14, wherein said degradation rate is less than about 6% per hour.

16. A rumen-protected lysine feed supplement comprising a lysine-producing bacteria strain having a rumen degradation rate of less than about 8% per hour as measured by the release of C$^{14}$ labelled leucine according to the method of Wallace et al.

17. The rumen-protected lysine feed supplement of aspect 14 or aspect 16, wherein the rumen degradation rate is such that more than 20% of the dosage of bacteria fed to a ruminant per day is delivered through the reticulo-rumen intact.

18. The rumen-protected lysine feed supplement of aspect 14 or aspect 16, wherein said bacteria strain is a *Corynebacteria glutamicum* ATCC strain selected from the group consisting 13058, 13825, 14066, 14067, 14068, 21127 and 700239.

19. A method for increasing the metabolically-available lysine content of a ruminant feed ration comprising adding to said feed ration an effective amount of the rumen-protected lysine feed supplement of aspect 10 or aspect 16.

20. The method of aspect 19, wherein said feed supplement is added to said feed ration in an amount effective to provide between about 5 and about 150 mg of metabolically available lysine per kg of ruminant body weight.

21. The method of aspect 19, wherein said ruminant is a dairy cow.

22. An in vitro method for evaluating the resistance of a lysine-producing bacteria strain to rumen inactivation in vivo, comprising the steps of:

culturing in vitro, a Gram positive lysine-producing bacteria strain in a nutrient medium containing natural or synthetic ruminal fluid; and
measuring the protein degradation in the bacteria culture as a function of time.

23. The method of aspect 22, wherein said bacteria strain is a strain of *Corynebacteria glutamicum.*

24. The method of aspect 23, wherein said *Corynebacteria glutamicum* strain is an ATCC strain selected from the group consisting 13058, 13825, 14066, 14067, 14068, 21127 and 700239.

25. The method of aspect 23, wherein said *Corynebacteria glutamicum* strain overproduces lysine.

26. The method of aspect 25, wherein said *Corynebacteria glutamicum* strain is genetically modified to overproduce lysine.

27. The method of aspect 22, wherein said protein degradation measuring step comprises measuring the release of C$^{14}$ labelled leucine according to the method of Wallace et al.

28. The method of aspect 22, wherein said method further includes the step of identifying as resistant to rumen inactivation bacterial strains having a degradation rate of less than 8% per hour.

29. The method of aspect 22, wherein said ruminal fluid is natural rumial fluid.

30. The method of aspect 22, wherein said ruminal fluid is synthetic ruminal fluid.

31. The method of aspect 28, wherein said method further includes the step of identifying as resistant to rumen inactivation bacteria strains having a rumen degradation rate such that more than 20% of the dosage of bacteria fed to a ruminant per day is delivered through the reticulo-rumen intact.

32. The method of aspect 31, wherein the identified strains have a degradation rate such that more than 50% of said dosage is delivered intact.

**Claims**

1.  A method for increasing the resistance to rumen inactivation of a lysine-producing Gram positive bacteria strain, the method comprising:

    growing a culture of the bacteria strain through at least one passage in a growth medium containing an amount of lysozyme effective to induce the growth of bacteria cell walls resistant to protozoal predation; and recovering the bacteria strain from the lysozyme-containing medium.

2.  A method according to claim 1, wherein the bacteria strain is a strain of *Corynebacteria glutamicum* or wherein the lysozyme concentration in the growth medium is between about 0.01 and about 100 ug/ml or between about 0.1 and about 10 ug/ml.

3.  A method according to claim 2, wherein:

    (a) the *Corynebacteria glutamicum* strain is an ATCC strain selected from the group consisting of 13058, 13825, 14066, 14067, 14068, 21127 and 700239; or
    (b) the *Corynebacteria glutamicum* strain overproduces lysine, for example is genetically modified to overproduce lysine.

4.  A method according to any preceding claim, wherein a plurality of passages in lysozyme-containing growth media are employed, such as between about 2 and about 10 passages are employed.

5.  A rumen-protected lysine feed supplement comprising a lysine-producing bacteria strain grown by the method of any preceding claim.

6.  A rumen-protected lysine feed supplement according to claim 5, wherein said bacteria strain is a strain of *Corynebacteria glutamicum,* optionally wherein the *Corynebacteria glutamicum* strain overproduces lysine, and preferably wherein the Corynebacteria glutamicum strain is genetically modified to overproduce lysine.

7.  A rumen-protected lysine feed supplement according to claim 5, wherein the bacteria strain has a rumen degradation rate of less than about 8 % per hour as measured by the release of $C^{14}$ labelled leucine according to the method of Wallace et al, optionally wherein the degradation rate is less than about 6 % per hour.

8.  A rumen-protected lysine feed supplement comprising a lysine-producing bacteria strain having a rumen degradation rate of less than about 8 % per hour as measured by the release of $C^{14}$ labelled leucine according to the method of Wallace et al.

9.  A rumen-protected lysine feed supplement according to any of claims 5 to 8 wherein the rumen degradation rate is such that more than 20% of the dosage of bacteria fed to a ruminant per day is delivered through the reticulorumen intact.

10. A rumen-protected lysine feed supplement according to any of claims 5 to 9 wherein said bacteria strain is a *Corynebacteria glutamicum* ATCC strain selected from the group consisting 13058, 13825, 14066, 14067, 14068, 21127 and 700239.

11. A method for increasing the metabolically-available lysine content of a ruminant feed ration, the method comprising adding to the feed ration an effective amount of the rumen-protected lysine feed supplement of any of claims 5 to 10.

12. A method according to claim 11, wherein the feed supplement is added to the feed ration in an amount effective to provide between about 5 and about 150 mg of metabolically available lysine per kg of ruminant body weight and/or wherein the ruminant is a dairy cow.

13. An in vitro method for evaluating the resistance of a lysine-producing bacteria strain to rumen inactivation in vivo, the method comprising:

    culturing in vitro, a Gram positive lysine-producing bacteria strain in a nutrient medium containing natural or synthetic ruminal fluid; and

measuring the protein degradation in the bacteria culture as a function of time; or

a lysine-producing bacterial strain grown by a method of any of claims 1 to 4.

14. A method according to claim 13, wherein:

(a) the bacteria strain is a strain of *Corynebacteria glutamicum*;

(b) the protein degradation measuring step comprises measuring the release of C[14] labelled leucine according to the method of Wallace et al;

(c) method further includes the step of identifying as resistant to rumen inactivation bacterial strains having a degradation rate of less than 8 % per hour;

(d) ruminal fluid is natural ruminal fluid;

(e) the ruminal fluid is synthetic ruminal fluid; or

(f) the method further includes the step of identifying as resistant to rumen inactivation bacteria strains having a rumen degradation rate such that more than 20% of the dosage of bacteria fed to a ruminant per day is delivered through the reticulo-rumen intact, such as wherein the identified strains have a degradation rate such that more than 50% of said dosage is delivered intact.

15. A method according to claim 14, wherein the *Corynebacteria glutamicum* strain is an ATCC strain selected from the group consisting of 13058, 13825, 14066, 14067, 14068, 21127 and 700239, or wherein the *Corynebacteria glutamicum* strain overproduces lysine, preferably wherein the *Corynebacteria glutamicum* strain is genetically modified to overproduce lysine.

FIG. 1

FIG. 2

FIG. 3

FIG. 4

# FIG. 5

## EUROPEAN SEARCH REPORT

Application Number

EP 10 01 2875

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MORGAVI D P ET AL: "Resistance of feed enzymes to proteolytic inactivation by rumen microorganisms and gastrointestinal proteases", JOURNAL OF ANIMAL SCIENCE, vol. 79, no. 6, June 2001 (2001-06), pages 1621-1630, XP002396174, ISSN: 0021-8812 | 13-15 | INV. C12N1/20 C12N1/36 A23K1/16 A23K1/18 C12Q1/04 ADD. C12R1/15 |
| A | * the whole document * | 1-12 | |
| X | VAN DE VYVER W F J ET AL: "Effect of glycosylation on the stability of fungal xylanase exposed to proteases or rumen fluid in vitro", ANIMAL FEED SCIENCE AND TECHNOLOGY, ELSEVIER, vol. 116, no. 3-4, 15 October 2004 (2004-10-15), pages 259-269, XP004573573, ISSN: 0377-8401 | 13-15 | |
| A | * abstract * | 1-12 | TECHNICAL FIELDS SEARCHED (IPC) |
| X | CHAVES ANTONIO HAMILTON ET AL: "Selection of isolates of Lactobacillus acidophilus used as probiotic for calves", REVISTA BRASILEIRA DE ZOOTECNIA, vol. 28, no. 5, September 1999 (1999-09), pages 1093-1101, XP009071416, | 13-15 | C12N A23K |
| A | * abstract * | 1-12 | |
| A | US 5 871 773 A (RODE ET AL) 16 February 1999 (1999-02-16) * the whole document * | 1-12 | |
| A | US 5 928 687 A (MEADE ET AL) 27 July 1999 (1999-07-27) * the whole document * | 1-12 | |

-/--

The present search report has been drawn up for all claims

| Place of search | Date of completion of the search | Examiner |
|---|---|---|
| Munich | 17 February 2011 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 01 2875

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SANTRA A ET AL: "Rumen manipulation to improve animal productivity.", ASIAN-AUSTRALASIAN JOURNAL OF ANIMAL SCIENCES, vol. 16, no. 5, May 2003 (2003-05), pages 748-763, XP009071415, ISSN: 1011-2367 * the whole document * | 1-12 | |

TECHNICAL FIELDS
SEARCHED      (IPC)

The present search report has been drawn up for all claims

| Place of search | Date of completion of the search | Examiner |
|---|---|---|
| Munich | 17 February 2011 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 10 01 2875

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2011

| Patent document cited in search report | | Publication date | Patent family member(s) | | Publication date |
|---|---|---|---|---|---|
| US 5871773 | A | 16-02-1999 | NONE | | |
| US 5928687 | A | 27-07-1999 | CA | 2140298 A1 | 15-07-1995 |

For more details about this annex : see Official Journal of the European Patent Office, No. 12/82

## REFERENCES CITED IN THE DESCRIPTION

### Patent documents cited in the description

- US 63361104 P
- US 4980164 A
- US 5256425 A
- US 6242230 B
- US 4562149 A, Shiozaki
- US 6737262 B, Bolla
- US 3959493 A
- US 3655864 A, Grass
- US 4473545 A, Drake
- US 4533557 A
- US 6238727 B
- US 5885610 A
- US 6013286 A, Klose
- EP 88166 A
- EP 387527 A
- EP 197335 A
- EP 219027 A
- EP 143195 A
- EP 358940 A
- DE 19831609
- DE 19548222

### Non-patent literature cited in the description

- **SIMILARLY, OHSUMI et al.** *Biosci. Biotech. Biochem.,* 1994, vol. 58, 1302-1305
- **STRAUSS et al.** *Can. J. Anim. Sci.,* 2004
- **TRYFONA et al.** *Process Biochem,* 2004
- **WALLACE et al.** *Br. J. Nutr,* 1987, vol. 58, 313-323
- **KUMAGAI.** *Bioscience, Biotechnology, and Biochemistry,* 2005, vol. 69, 2051-2056
- **KEILHAUER et al.** *J Bacteriol,* 1993, vol. 175, 5595-5603
- **LENNOX, E. S.** *Virology,* 1955, vol. 1, 190-206
- **BROER ; KRAMER.** *J. Bacteriol.,* 1990, vol. 172, 7241-7248
- **SCOTT ; DEHORITY.** *J. Bacteriol.,* 1965, vol. 89, 1169-1175
- **HOBSON.** *Methods Microbiol.,* 1969, vol. 3B, 133-149
- **WALLACE.** *Int. J. Syst. Evol. Microbiol.,* 2003, vol. 53, 965-970
- **HUNGATE R E.** The rumen and its microbes. Academic Press, 1966
- **HOBSON ; STEWART.** *The Rumen Microbial Ecosystem*
- **LVAN.** *J Anim Sci,* 2000, vol. 78, 750-759
- **LVAN.** *J Dairy Sci,* 2000, vol. 83, 776-787
- **WALLACE et al.** *Br. J. Nutr.,* 1987, vol. 58, 313-323